# EUROPEAN PATENT SPECIFICATION

(11) **EP 1 071 508 B1**
(45) Date of publication and mention of the grant of the patent: **04.08.2010**
(21) Application number: 99914152.6
(22) Date of filing: 25.03.1999
(51) Int. Cl.: B01J 23/89, C07C 5/333

(54) **DEHYDROGENATION CATALYSTS COMPRISING AT LEAST IRON, ALKALI METAL AND A NOBLE METAL**
ZUMINDEST EISEN, ALKALIMETALL UND EDELMETALL ENTHALTENDE DEHYDRIERUNGSKATALYSATOREN
CATALYSEURS DE DESHYDROGENATION COMPORTANT AU MOINS DU FER, UN METAL ALCALIN ET UN METAL NOBLE

(30) Priority: 01.04.1998 US 53234; 26.01.1999 US 237408
(43) Date of publication of application: 31.01.2001
(73) Proprietor: Süd-Chemie Inc., Louisville Kentucky 40232-2370 (US); Süd-Chemie Catalysts Japan, Inc., Tokyo (JP)
(72) Inventor: WILLIAMS, David, Louisville, Kentucky 40272 (US); MISHIMA, Yuji Sud-Chemie Nissan Catalysts Inc., Chiyodaku Tokyo 102-0074 (JP); ROKICKI, Andrzej, Prospect, Kentucky 40059 (US); SHINYAMA, Kazuhiko Sud-Chemie Nissan Catalysts Inc, Chiyodaku Tokyo 102-0074 (JP); SMITH, Dennis, Louisville, Kentucky 40299 (US)
(74) Representative: Heinemann, Monica
(86) International application number: PCT/US1999/006603
(87) International publication number: WO 1999/049968

(56) References cited:
- DE-A- 4 303 715
- US-A- 3 424 808
- US-A- 3 798 178
- US-A- 3 904 552
- US-A- 5 023 225
- DATABASE WPI Section Ch, Week 7447 Derwent Publications Ltd., London, GB; Class A41, AN 74-81878V XP002108632 & JP 49 039973 B (TORAY IND INC), 30 October 1974 (1974-10-30)
- DATABASE WPI Section Ch, Week 9726 Derwent Publications Ltd., London, GB; Class A41, AN 97-289096 XP002108633 & WO 97 18034 A (CHINA PETROCHEMICAL TECHNOLOGY CO), 22 May 1997 (1997-05-22)

## Description

### Background of the Invention

The field of art to which this invention pertains is dehydrogenation catalysts.

In the catalytic dehydrogenation of alkylaromatic hydrocarbons to alkylaromatic hydrocarbons, e.g., the dehydrogenation of ethylbenzene to styrene, considerable efforts have been expended to develop catalysts which exhibit high conversion combined with high selectivity and increased stability.

Promoted iron oxide catalysts have been found to be especially useful in the dehydrogenation of alkylaromatic hydrocarbons to alkylaromatic hydrocarbons. Typical commercial iron oxide-based dehydrogenation catalysts are generally promoted with the addition of other metal compounds, in the form of, but not limited to, oxides, hydroxides, carbonates, nitrates, etc. Often one of the promoters is an alkali metal compound with potassium being preferred. Other components may also be added to the dehydrogenation catalyst to provide further promotion, activation or stabilization. In all such dehydrogenation catalysts, minor amounts of modifiers are also typically present, such as organic burn-out agents: carbon black, graphite, methylcellulose, etc., which can beneficially effect the pore structure and/or other physical properties of the catalyst. In the discussion of the different metal groups, the reference will be based on the new IUPAC notation of the periodic table.

Typical catalysts used in dehydrogenation of saturated hydrocarbons to unsaturated hydrocarbons, as disclosed in U.S. patent No. 2,866,790, are iron oxide catalysts containing a small amount of chromium oxide as a stabilizer and a small amount of potassium compound as promoter. Improved catalysts according to this patent are made from iron oxide (39 to 47 weight percent), chromium oxide (1 to 10 weight percent), and potassium carbonate (51 to 59 weight percent).

Dehydrogenation catalysts having good physical strength are described in U.S. Patent No. 2,866,791. These catalysts are made from 51 to 59 weight percent potassium fluoride, 1.0 to 10 weight percent chromium oxide with the balance being iron oxide (39 to 47 weight percent).

Catalysts designed for the dehydrogenation of alkylbenzenes, at elevated temperatures in the presence of steam, comprising iron oxide and as a promoter from about 1 to about 25 percent by weight of an alkali metal oxide, from about 1 to about 10 percent by weight of a rare earth metal oxide, and from about 0.1 to about 10 percent by weight calcium oxide, are disclosed in U.S. Patent No. 4,749,674.

Another catalyst for the dehydrogenation of ethylbenzene to styrene disclosed in U.S. Patent No. 5,510,552 contains at least one iron oxide, at least one bicarbonate, oxide or hydroxide of potassium and/or cesium, an oxide, carbonate, nitrate or hydroxide of cerium, a hydraulic cement, from about 0.2 to about 10 percent of a sodium oxide and from about 1.5 to about 20 percent calcium oxide.

WO 96/18458 discloses a method of preparing an iron oxide catalyst comprising contacting an iron oxide with a additive comprising an element selected from a large group of elements on the periodic chart, heating that iron oxide mixture to a temperature of at least about 600°, to afford structural rearrangement of the particle habit of said iron oxide, and then forming it into the catalyst. See also WO 96/18594 and WO 96/18593.

Similarly, U.S. Patent No. 5,668,075 discloses the preparation of improved selectivity iron oxide dehydrogenation catalysts based on reconstructed iron oxides. The reconstruction of the oxides comprises contacting an iron oxide with a dopant substance comprising elements selected from a large group of components of the periodic chart and heating the doped iron oxide to a temperature of at least about 600°C, preferably 800°C and 1100°C. As in the previous references, rearrangement of particle habit is induced in iron oxide prior to it being formed into catalyst. Metal additives, disclosed in the teachings of the patent, are solely and specifically used to promote the physical transformation of the iron oxide and not the chemical properties of the catalyst formed based on the oxide.

Another dehydrogenation catalyst, which contains smaller amounts of iron oxide and relatively larger amounts of cerium oxide and potassium carbonate, is disclosed in U.S. Patent No. 4,758,543. Catalysts having good activity and good selectivity are described in U.S. patent No. 3,904,552. These catalysts are made with iron oxide and alkali metal oxides plus molybdenum oxide and cerium oxide. Similar catalysts utilizing tungsten oxide in place of molybdenum oxide are described in U.S. Patent No. 4,144,197.

Dehydrogenation catalysts which maintain high activity and selectivity over extended periods of time are described in U.S. Patent No. 4,467,046. These catalysts contain iron oxide, an alkali metal compound, a cerium compound, a molybdenum compound and a calcium compound.

Improving stability of Fe/K/Ce/Mo/Ca/Mg oxide catalysts by incorporation of small amounts of chromium (100 to 5000 ppm) into the iron oxide prior to forming the catalyst is taught in U.S. Patent No. 5,023,225.

The addition of titanium also results in improved activity and selectivity of iron oxide/potassium oxide catalytic systems, for ethylbenzene to styrene dehydrogenation, according to U.S. Patent No. 5,190,906.

Dehydrogenation catalysts made from iron oxide, chromium oxide and kaolinite plus potassium oxide are disclosed in U.S. Patent No. 4,134,858. The catalysts can also contain at least one oxide of copper, vanadium, zinc, magnesium, manganese, nickel, cobalt, bismuth, tin, or antimony.

U.S. Patent Nos. 3,424,808 and 3,505,422 are directed to dehydrogenation catalysts which consist essentially of iron oxide, a minor amount of an alkali metal hydroxide or carbonate, and a minor amount of transition metal, preferably ruthenium, cobalt, or nickel.

Catalysts for the dehydrogenation of para-ethyltoluene to para-methylstyrene are described in U.S. Patent Nos. 4,404,123; 4,433,186; 4,496,662; and 4,628,137. These catalysts are made with iron oxide and potassium carbonate, plus chromic oxide, gallium trioxide, or magnesium oxide. Each patent also discloses that the catalysts can optionally contain compounds of cobalt, cadmium, aluminum, nickel, cesium, and rare earth elements as stabilizers, activators and promoters. Other dehydrogenation catalysts and procedures for their use and manufacture are shown in U.S. Patent Nos. 2,408,140; 2,414,585; 3,360,579; 3,364,277; and 4,098,723.
WO97/18034 discloses a dehydrogenation catalyst for producing unsaturated aromatics **characterized in that** said catalyst comprises Fe₂O₃ 40∼70%, K₂O 10∼40%, MnO₃ 0∼5%, MgO 0.05∼5% and 0.001∼5% at least one oxide of the elements selected from the group consisting of Cu, Zn, Sc, Ti, W, Mn, Ni, Pd, Al, P, Bi, B, Sn, Pb and Si by weight, and that the catalyst comprises further at least two compounds of rare-earth metal elements, the content of which is from 2% to 15% on the basis of the oxides.

Dehydrogenation reactions are normally conducted at the highest practical throughput rates to obtain optimum yield. Yield is dependent upon conversion and selectivity of the catalyst.

Selectivity of the catalyst is defined as the proportion of the desired product, e.g., styrene, produced to the total amount of feedstock, e.g., ethylbenzene, converted. Activity or conversion is that portion of the feedstock which is converted to the desired product and by-products.

Improvements in either selectivity or activity can result in substantially improved operating efficiency. Higher activity catalysts, for example, would allow operation at lower temperatures than currently available catalysts, for any given conversion. Thus, in addition to high energy efficiency, the catalyst would be expected to last longer and generate less thermal by-products.

The ratio of benzene to toluene, B/T ratio, in the final product is another criteria to be used in determining effectiveness of the catalyst. The benzene by-product produced can be recycled for later processing. Toluene can not be easily recycled and is considered an undesirable by-product. Thus catalysts yielding higher B/T by-product ratio, all other factors the same, will be preferred.

There is thus a need for a dehydrogenation catalyst which has good selectivity and activity.

It is, therefore, an object of the invention to provide a novel dehydrogenation catalyst.

It is another object of the invention to provide an improved dehydrogenation catalyst having both high activity and selectivity.

It is another object of this invention to provide an improved catalyst for the conversion of ethylbenzene to styrene, with high activity and high selectivity.

It is another object of the invention to provide an improved dehydrogenation catalyst containing at least iron oxide, an alkali metal oxide, and a noble metal as a promoter as defined below.

It is still a further object of this invention to provide an improved process for the production of olefinic compounds, particularly styrene. These and other objects are obtained by the product and process of the present invention.

This invention is directed to an improved dehydrogenation catalyst according to claim 1, preferably for use in the dehydrogenation of ethylbenzene to styrene.

A dehydrogenation catalyst according to the invention for an alkylaromatic feed stream containing steam comprises from 40 to 90 weight percent iron oxide calculated as Fe₂O₃, from 5 to 20 weight percent of an alkali metal compound calculated as an alkali metal oxide, from 1 ppm to 100 ppm of a source of noble metal source selected from the group consisting of an elemental noble metals, compounds containing a noble metal and combinations thereof, wherein the noble metal is selected from the group consisting of platinum, palladium and combinations thereof, from 0.5 to 10.0 weight percent of a molybdenum or tungsten compound, calculated as MoO₃ or WO₃ and from 4.0 to 12.0 weight percent of a cerium compound, calculated as CeO₂, wherein all weight percents are based on the total weight of the catalyst.

For palladium the amount of metal present can be less than 100 ppm, and preferably also less than 20 ppm.

The invention is also directed to a process for the production of olefinic compounds by dehydrogenation, utilizing the catalyst of the invention according to claim 2. The invention is preferably an improved process for the production of styrene from ethylbenzene utilizing the inventive catalyst.

### Brief Description of the Drawing

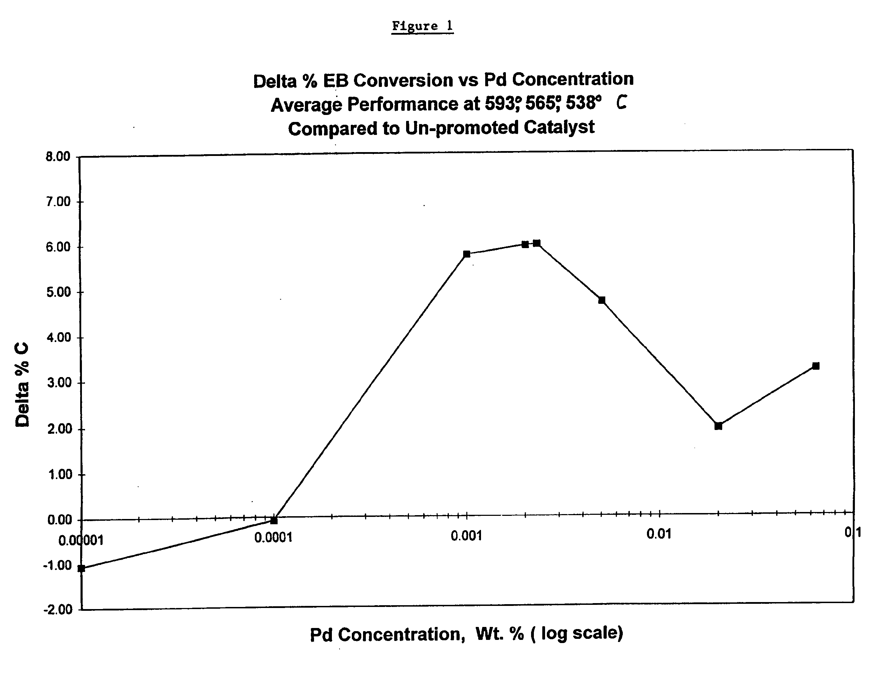

Figure 1 shows the effect of the amount of palladium loading on the performance of a catalyst. It shows the average difference in ethyl benzene conversion between catalyst with and without various concentrations of palladium on the catalyst body.

### Description of the Invention

The catalysts of this invention are made by combining an iron compound, such as iron oxide or a ferrite, preferably potassium ferrite, with an alkali metal source, which can be in the form of, but is not limited to, oxides, hydroxides, carbonates, nitrates or bicarbonates, preferably a sodium or potassium derivative, and most preferably potassium carbonate, and a source for a noble metal. The source for the noble metal may include elemental noble metal, compounds containing the noble metal or combinations thereof. The term "noble metal" includes platinum, palladium, rhodium, ruthenium, rhenium, iridium and osmium, wherein platinum and palladium are used according to the invention.

In addition to the above-described components, the catalyst also includes as promoters an oxide or salt of cerium. The catalyst also includes molybdenum or tungsten compounds, preferably oxides, most preferably molybdenum oxide. The catalyst containing palladium preferably also includes alkaline earth metal compounds, most preferably magnesium oxide or calcium oxide. The catalyst may then also include a source for titanium, chromium or silicon or aluminum, preferably an oxide or salt. The catalyst may then also include a source for at least one of the following elements including zinc, manganese, copper, cobalt and vanadium and combinations thereof.

For palladium, the amount of metal can also be less than 100 ppm, or less than 20 ppm. Additional components of the catalyst may then include from 50 ppm to 4.0 weight percent of chromium oxide calculated as Cr₂O₃ and from 10 ppm to 2000 ppm of titanium oxide calculated as TiO₂. The catalyst may then also include from 0.1 to 10.0 weight percent of the salt or oxide of one or more of the following: aluminum, silicon, zinc, manganese, cobalt, cadmium, vanadium and copper, alone or in combination, calculated on an elemental basis.

The dehydrogenation catalyst according to the invention contains from 40 to 90 weight percent iron oxide calculated as Fe₂O₃, from 5 to 20 weight percent of an alkali metal compound calculated as an alkali metal oxide and from 1 ppm to 100 ppm of a source of a noble metal, wherein the noble metal is palladium and/or platinum. The source for the noble metal is selected from the group including elemental palladium, elemental platinum, compounds containing palladium and/or platinum. The catalyst also includes from 0.5 to 10.0 weight percent of a molybdenum or tungsten compounds calculated as MoO₃ or WO₃, and from 4.0 to 12.0 weight percent of a cerium compound, calculated as CeO₂, wherein all weight percents are based on the total weight of the catalyst. Additional promoters may be included with this palladium containing catalyst as discussed above.

A most preferable dehydrogenation catalyst contains from 40 to 90 percent iron oxide calculated as Fe₂O₃, 5 to 20 percent of an alkali metal compound, preferably potassium oxide, 4.0 to 12 percent of cerium oxide calculated as CeO₂, 0.5 to 10.0 percent of molybdenum or tungsten oxide calculated as MoO₃ or WO₃, preferably molybdenum oxide, 0.2 to 10.0 percent of calcium or magnesium oxide, preferably calcium oxide, 10 ppm to 1000 ppm of titanium oxide calculated as TiO₂, 100 ppm to 2000 ppm of chromium oxide calculated as Cr₂O₃, and less than 20 ppm of a source for a noble metal, wherein the noble metal is palladium, and wherein the percentage is calculated on an elemental basis. Additional components that can be added to this catalyst include from about 0.1 to about 10.0 weight percent of an oxide of aluminum, silicon, manganese, copper, zinc, cadmium, vanadium, and cobalt, calculated on an elemental basis.

It is advantageous to prepare the catalyst using one or a combination of the following methods: co-precipitation, decomposition, impregnation and mechanical mixing or any other method, as would be readily appreciated by those skilled in the art. The method chosen should guarantee intimate mixing and uniform distribution of the components.

It is well established in the art that different forms of iron oxide, red, yellow, brown and black, can be used for preparation of the dehydrogenation catalyst. Likewise, it is known in the art that the iron oxides can be derived from a variety of precursor materials, both natural and synthetic, using a number of processes. Generally, iron is added to the catalyst compositions as red iron oxide, Fe₂O₃, or yellow iron oxide, Fe₂O₃·H₂O, but others can be readily utilized as would be appreciated by those skilled in the art. Particularly suited are pigment grades of the iron oxides. Ferrites may also be used, such as potassium ferrite.

Likewise, the Pd-containing catalyst promoter can be any material taught by the art. Potassium compounds are the preferred alkali metal promoters. The promoter can be added to the catalyst in various forms. Alkali metal oxides, hydroxides, carbonates, bicarbonates, and the like, and mixtures thereof are preferred, with potassium carbonate or a mixture of potassium carbonate with potassium oxide is most preferred.

The catalyst compositions of the present invention also contain compounds of cerium to enhance conversion and/or selectivity depending on the co-promoters. Cerium, used in the catalyst compositions of the present invention, can be added to the catalyst in the form of cerium oxide or in the form of other cerium compounds, as for example, cerium carbonate, cerium nitrate, cerium hydroxide, or any combination thereof.

Other known catalyst additives can be included in the Pd-containing catalysts of the present invention, but are not essential. A chromium compound, which can serve as a stabilizer for the active catalytic components, is illustrative of an optional, but preferred, additive. Chromium compounds are added to alkali-promoted iron oxide catalysts to extend their life and improve stability at low steam to oil conditions of operation. Chromium, as used in the compositions of the present invention, can be added to the catalyst in the form of a chromium oxide or in the form of a chromium salt. Preferably, chromium is added by spiking of the iron oxide used in catalyst preparation as taught in U.S. Patent No. 5,023,225.

The addition of titanium is taught in U.S. Patent No. 5,190,906. Other components, used to improve selectivity of the catalyst, include molybdenum or tungsten, which can be added as respective oxides or salts, including derivatives of corresponding oxo acids (i.e. molybdates or tungstates, respectively). In addition, a number of other metal compounds may be added as promoters. These can include, but are not limited to, compounds of aluminum, vanadium, cobalt, cadmium, copper, calcium, magnesium, and manganese.

The physical strength, activity and selectivity of the catalyst compositions of the present invention can be improved by adding certain binding agents. Binding agents can include, but are not limited to, hydraulic cements, calcium aluminate or Portland cement. These agents can be added individually or in combination.

The density of the catalyst composition can be modified by the addition of various filler substances, for example, combustible materials such as graphite and methyl cellulose. Such materials can be added to the compositions during preparation, but are burned out after the catalyst pellets have been formed during the calcining step. Porosity promoting aids can also facilitate extrusion of catalyst pellets.

The catalyst components can be mixed in various ways known to the art. One method comprises ballmilling together a mixture of desired compounds, adding a small amount of water, and extruding the composite to produce small pellets, which are then dried and calcined. Another method is mixing the components together with water, drying them to form a powder, and tableting and calcining the tablets. Another procedure involves mixing the components together with an excess of water, partially drying, and then subsequently extruding, drying, and calcining the resulting pellets. The choice of the mixing method depends on the preference of the skilled artisan.

A preferred method of preparing the catalyst is to blend the catalyst ingredients together in the presence of sufficient water to make a moist extrudable mixture. This mixture is then extruded to produce extrudates of desired shape and size, typically cylindrical pellets having a diameter of about 3 mm. The extrudates are then calcined under conventional calcining conditions. Calcination temperatures can range from about 500°C to about 1200°C, preferably from about 600°C to about 1000°C. After calcination, the extrudates are ready for use as catalyst.

Known methods can be used to form the catalyst mass. Preferred forming methods are pelletizing, extruding and tableting, in which the use of inorganic or organic auxiliaries as lubricants to improve plasticity during extrusion is recommended. Forming can also be undertaken both before and after calcination.

The efficacy of the noble metal addition is independent of the method of addition or the point in the manufacturing process at which it is incorporated. The following are some methods for delivery of the noble metal promoter. A number of alternative methods would be obvious to one skilled in the art.

The noble metal, palladium and/or platinum, additives can be directly added to the iron oxide and the mixture can be pre-fired at about 300°C to about 500°C prior to blending with the other components. Alternatively, the noble metal, palladium and/or platinum, can be co-precipitated with iron oxide prior to the iron oxide being blended. The noble metal, palladium and/or platinum additives can be impregnated onto the surface of the finished catalyst followed by drying and re-calcination at a temperature adequate to drive-off water and decompose the impregnated salt. However, addition of the noble metal, palladium and/or platinum, metal additives in the form of an aqueous solution of appropriate salts, preferably nitrates, directly to the catalyst blend, immediately prior to mulling and pelletizing, is preferred.

Heat treatment or calcination can be conducted under static conditions, for example, in a tray furnace, or under dynamic conditions, such as in a rotary kiln. The temperatures and residence times are determined for each individual type of catalyst.

The catalysts preferably occur as moldings, especially in the form of spheres, pellets, rings, tablets or extruded products, in which they are formed as solid or hollow objects in order to achieve a high geometric surface with a simultaneously low resistance to flow.

The BET surface area of the catalysts is typically about 0.5 to about 12 m²/g, and preferably, about 1.5 to about 4 m²/g. The BET surface is determined by N₂ adsorption, as described in ASTM D3663-92.

The specific pore volume is determined according to the mercury penetration method described in J. Van Brakel, et al., Powder Technology, 29, p.1 (1981). In this method, mercury is pressed up to a pressure of about 4000 bar into the catalyst moldings, during which the volume reduction of the mercury is plotted as a function of pressure. A curve is obtained from which the pore distribution can also be determined. According to this mercury penetration method, only the volume and distribution of pores with a diameter of >3.6 nm can be determined. Generally, catalysts with larger pore volume and higher median pore diameter are preferred as taught in U.S. Pat No. 5,689,023. Typical pore volume of the catalysts of the present invention is in the range of ca. 0.10 to 0.45 cc/g.

One skilled in the art will readily appreciate that surface area, total pore volume and pore volume distribution can be adjusted with proper manufacturing techniques to get optimum performance for any given catalyst composition. This not withstanding, the promotional effect of the noble metal, addition to the formulations will still be unmistakable.

The catalysts of the present invention are effective as dehydrogenation catalysts and especially effective in promoting the dehydrogenation of ethylbenzene to produce styrene. Such dehydrogenation reactions are generally carried out at reaction temperatures from about 480°C to about 700°C, preferably about 535°C to about 650°C. The use of subatmospheric, atmospheric, or superatmospheric pressures are suitable for the reactions. However, based on equilibrium and selectivity considerations, it is preferred to operate at as low a pressure as is feasible. Therefore, atmospheric or subatmospheric pressure is preferred. Typically the dehydrogenation process using the catalysts of this invention is conducted as a continuous operation utilizing a fixed bed which may consist of a single stage or a series of stages of the same or different catalysts in one or more reactors. Other types of reactors and reactor configurations can be used for the dehydrogenation process.

In the dehydrogenation process using the catalyst of this invention, steam is added to the hydrocarbon feedstock to aid in the removal of carbonaceous residues from the catalyst and to furnish heat for the reaction. Steam to hydrocarbon molar ratios from 3 to 18 or higher can be used. However, in order to conserve energy in the operation of the process, steam to hydrocarbon molar ratios (S/O) of 12 or lower are preferred.

The contact time of the reactant-containing gas with the catalyst is expressed in terms of liquid-hourly-space velocity (LHSV) which is defined as the volume of liquid hydrocarbon reactant per volume of catalyst per hour. The LHSV of the organic reactants can vary between 0.1 hour⁻¹ and 5 hour⁻¹.

It has been surprisingly discovered that extremely small quantities of noble metals when added to the catalyst result in enhanced performance and selectivity. In fact, it has been surprisingly discovered that smaller quantities of the noble metals are more effective than larger quantities of noble metals (greater than 5000 ppm). For palladium according to the invention, the amount of metal present is preferably less than 100 ppm, and most preferably less than 20 ppm. It has also been surprisingly discovered that extremely small quantities perform as well as larger quantities, such as quantities greater than about 1000 ppm.

When used in the continuous process of dehydrogenating ethyl benzene to styrene, the catalysts of this invention exhibit better performance, i.e. higher conversion, improved yield and higher B/T ratio, than similar catalysts which do not contain noble metals.

### EXAMPLES

The following examples describe the invention in more detail. Parts and percentages are by weight unless otherwise designated. Iron oxide used in all the following preparations is a commercial product that may contain ppm levels of Ti and Cr and may also contain minor amounts of other elements such as Si, Al, Mn, Mg, S, Cl, Zn, V, Cu, etc.

### Comparative Example 1

Comparative dehydrogenation catalyst 1, with a composition of 11.2% potassium oxide (K₂O), 88.8% iron oxide

(Fe₂O₃) was prepared as follows:
A mixture of the required amounts of potassium carbonate and unhydrated iron oxide were dry blended with a small amount of organic lubricant/poreformer, mixed with water to form an extrudable paste and then formed into cylindrical pellets of 3 mm diameter. The pellets were dried several hours and then calcined (at 600°C).

### Comparative Example 2

The catalyst of Comparative Example 2 was prepared according to the procedure of Comparative Example 1, except that a palladium nitrate solution sufficient to produce a concentration of 0.072% Pd in the final catalyst was added to the water used to prepare the extrudable paste.

The catalysts of Comparative Example 1 and Comparative Example 2 were tested for ethylbenzene dehydrogenation performance in an externally heated tubular reactor of 1" internal diameter. A vaporized, preheated mixture of steam and ethylbenzene (with a molar ratio of about 12/1) was introduced to the catalyst at controlled throughput and pressure (LHSV = 1 and pressure = 1 atm.) over a range of temperature from 540°C to 570°C. Dehydrogenated product exiting the reactor was collected and analyzed to determine conversion (%C) of ethylbenzene and selectivity (%S) to styrene. Table I shows the effect on performance of the catalysts.

**TABLE I**

| Catalyst | Comparative Example 1 | | Comparative Example 2 | |
|---|---|---|---|---|
| Palladium | | | | |
| concentration wt.% | -- | | 0.072 | |
| Dehydrogenation | | | | |
| Performance | %C | %S | %C | %S |
| 570°C | 46.86 | 94.62 | 50.10 | 94.27 |
| 540°C | 25.62 | 96.15 | 33.30 | 96.04 |

### Comparative Example 3

The dehydrogenation catalyst of Comparative Example 3 having the following nominal composition on oxide basis:

| | |
|---|---|
| 9.89% | K₂O |
| 9.97% | CeO₂ |
| 2.53% | MoO₃ |
| 77.61% | Fe₂O₃ |

was prepared as follows:
A mixture of the required amounts of potassium carbonate, cerium carbonate, molybdenum oxide, and unhydrated iron oxide were dry blended with a small amount of organic lubricant/poreformer, mixed with water to form an extrudable paste, and then formed into cylindrical pellets of 3 mm diameter. The pellets were dried several hours and then calcined at 900°C.

### Comparative Examples 4 and 5

The catalysts of Comparative Examples 4 and 5 were prepared according to the procedure for the catalyst of Comparative Example 3 except that palladium nitrate solution, Comparative Example 4, or dinitrodiamine platinum solution, Comparative Example 5, sufficient to produce a concentration of 200 ppm palladium or 368 ppm platinum in the respective calcined catalysts, was added to the water used to prepare the extrudable paste for pelletizing the catalysts.

The catalysts of Comparative Example 3 and Comparative Examples 4 and 5 were granulated (to a size of 0.85 to 1.18 mm) and evaluated for ethylbenzene dehydrogenation performance in a differential type reactor (steam/oil=12 molar, p=1 atm., catalyst weight/feed rate=14.7 times (g. cat. x hr. ÷ mol). Dehydrogenation performance data are shown in Table II along with the indicated concentration of Pd or Pt. As in Comparative Example 1, catalyst performance was determined by analysis of the dehydrogenated product exiting the reactor.

**TABLE II**

| | Comparative Example 3 | | Comparative Example 4 | | Comparative Example 5 | |
|---|---|---|---|---|---|---|
| Promoter | none | | Pd | | Pt | |
| Promoter Concentration wt. % | ---- | | 0.0200 | | 0.0368 | |
| D.P.* | %C | %S | %C | %S | %C | %S |
| 600°C | 30.73 | 98.41 | 35.94 | 98.21 | 35.54 | 98.51 |
| 585°C | 21.60 | 98.77 | 28.56 | 98.63 | 27.59 | 98.90 |
| 570°C | 14.23 | 98.96 | 21.94 | 98.89 | 21.41 | 99.13 |
| 555°C | 9.15 | 99.09 | 16.32 | 99.08 | 16.15 | 99.25 |
| 540°C | 5.54 | 99.13 | 11.72 | 99.20 | 12.16 | 99.32 |

| | | | | | | |
|---|---|---|---|---|---|---|
| *Dehydrogenation Performance | | | | | | |

### Comparative Example 6

The dehydrogenation catalyst of Comparative Example 6 having the following nominal composition, on oxide basis:

| | |
|---|---|
| 9.89% | K₂O |
| 9.97% | CeO₂ |
| 2.53% | WO₃ |
| 77.61% | Fe₂O₃ |

was prepared as follows:
A mixture of the required amounts of potassium carbonate, cerium carbonate, tungsten oxide, and unhydrated iron oxide were dry blended with a small amount of organic lubricant/poreformer, mixed with water to form an extrudable paste, and then formed into cylindrical pellets of 3 mm diameter. The pellets were dried several hours and then calcined at 900°C.

### Comparative Examples 7 and 8

The catalysts of Examples 7 and 8 were prepared according to the procedure for the catalyst of Comparative Example 6 except that palladium nitrate solution, Example 7, or dinitrodiamine platinum solution, Example 8, sufficient to produce a concentration of 200 ppm palladium or 368 ppm platinum in the respective calcined catalysts, was added to the water used to prepare the extrudable paste for pelletizing the catalysts.

The catalysts of Comparative Example 6 and Comparative Examples 7 and 8 were granulated (to a size of 0.85 to 1.18 mm) and evaluated for ethylbenzene dehydrogenation performance in the manner described in Comparative Example 3. Dehydrogenation performance data are shown in Table III along with the indicated concentration of Pd or Pt.

**TABLE III**

| | Comparative Example 6 | | Comparative Example 7 | | Comparative Example 8 | |
|---|---|---|---|---|---|---|
| Promoter | none | | Pd | | Pt | |
| Promoter Concentration wt. % | --- | | 0.0200 | | 0.0368 | |
| D.P.* | %C | %S | %C | %S | %C | %S |
| 600°C | 30.18 | 98.55 | 43.04 | 97.87 | 37.12 | 98.58 |
| 585°C | 21.10 | 98.89 | 34.60 | 98.43 | 28.79 | 98.95 |
| 570°C | 14.39 | 99.05 | 27.06 | 98.82 | 22.16 | 99.15 |
| 555°C | 9.49 | 99.15 | 20.65 | 99.00 | 16.78 | 99.28 |
| 540°C | 5.91 | 99.19 | 14.99 | 99.17 | 12.74 | 99.37 |

| | | | | | | |
|---|---|---|---|---|---|---|
| *Dehydrogenation Performance | | | | | | |

### Comparative Example 9

The dehydrogenation catalyst of Comparative Example 9 having the following nominal composition, on oxide basis:

| | |
|---|---|
| 9.5% | K₂O |
| 2.2% | MgO |
| 5.0% | CeO₂ |
| 2.5% | MoO₃ |
| 2.0% | CaO |
| 78.8% | Fe₂O₃ |

was prepared as follows:
A mixture of the required amounts of potassium carbonate, magnesium carbonate, cerium carbonate, molybdenum oxide, calcium hydroxide and unhydrated iron oxide were dry blended with a small amount of organic lubricant/poreformer, mixed with water to form an extrudable paste, and then formed into cylindrical pellets of 3 mm diameter. The pellets were dried several hours and then calcined (at 600°C).

### Comparative Examples 10, 11, 12 and Example 13

The catalysts of (Comparative) Examples 10, 11, 12 and 13 were prepared according to the procedure for the catalyst of Comparative Example 9 except that amounts of a palladium nitrate solution, sufficient to produce the target concentration of palladium in the calcined catalyst, were added to the water used to prepare the extrudable paste for pelletizing.

The catalysts of Comparative Example 9 and (Comparative) Examples 10 through 13 were granulated (to a size of 0.85 to 1.18 mm) and evaluated for ethylbenzene dehydrogenation performance in the manner described in Comparative Example 3. Dehydrogenation performance data are shown in Table IV along with the indicated concentration of Pd.

**TABLE IV**

| | Comparative Example 9 | | Comparative Example 10 | | Comparative Example 11 | | Comparative Example 12 | | Example 13 | |
|---|---|---|---|---|---|---|---|---|---|---|
| Palladium Concentration wt. % | ---- | | 0.12 | | 0.064 | | 0.031 | | 0.010 | |
| D.P.* | %C | %S | %C | %S | %C | %S | %C | %S | %C | %S |
| 600°C | 34.8 | 98.4 | 44.5 | 98.0 | 43.1 | 97.8 | 40.8 | 97.9 | 47.7 | 97.3 |
| 585°C | 24.1 | 98.9 | 36.4 | 98.5 | 34.7 | 98.3 | 33.0 | 98.3 | 39.2 | 98.1 |
| 570°C | 16.2 | 99.1 | 28.6 | 98.7 | 26.6 | 98.4 | 25.7 | 98.5 | 30.9 | 98.6 |
| 555°C | 10.3 | 99.1 | 21.4 | 98.9 | 19.4 | 98.5 | 19.0 | 98.8 | 23.5 | 98.9 |
| 540°C | 6.4 | 99.1 | 15.8 | 98.9 | 13.5 | 98.5 | 13.5 | 98.9 | 17.0 | 99.2 |

| | | | | | | | | | | |
|---|---|---|---|---|---|---|---|---|---|---|
| *Dehydrogenation Performance | | | | | | | | | | |

### Comparative Examples 14, 15, 16, 17

The catalysts of Comparative Examples 14, 15, 16 and 17 were prepared according to the procedure of Comparative Example 9 except that amounts of ruthenium nitrate, chloroiridic acid or rhodium chloride, sufficient to produce the indicated concentrations of each noble metal promoter in the final catalyst, were added to the water used to prepare the extrudable paste.

The catalyst of Comparative Example 9 and Examples 14, 15, 16 and 17 were granulated (to a size of 0.85 to 1.18 mm) and tested in the manner described in Comparative Example 3. Dehydrogenation performance data are shown in Table V along with the respective concentrations of Ru, Ir or Rh in each catalyst.

**Table V**

| | Comparative Example 9 | | Comparative Example 14 | | Comparative Example 15 | | Comparative Example 16 | | Comparative Example 17 | |
|---|---|---|---|---|---|---|---|---|---|---|
| Promoter | None | | Ru | | Ir | | Rh | | Rh | |
| Promoter Concentration wt. % | ---- | | 0.02 | | 0.005 | | 0.001 | | 0.01 | |
| D.P.* | %C | %S | %C | %S | %C | %S | %C | %S | %C | %S |
| 600 Deg. C | 34.8 | 98.4 | 36.33 | 98.52 | 37.65 | 98.4 | 40.48 | 98.28 | 42.19 | 97.75 |
| 585 Deg. C | 24.1 | 98.9 | 27.65 | 98.9 | 27.42 | 98.86 | 30.41 | 98.77 | 33.99 | 98.01 |
| 570 Deg. C | 16.2 | 99.1 | 20.73 | 90.09 | 19.32 | 99.08 | 22.23 | 99.02 | 26.08 | 98.14 |
| 555 Deg. C | 10.3 | 99.1 | 15.23 | 99.21 | 13.3 | 99.2 | 15.73 | 99.18 | 19.52 | 98.36 |
| 540 Deg. C | 6.4 | 99.1 | 10.74 | 99.29 | 8.83 | 99.29 | 10.88 | 99.27 | 14.48 | 98.65 |

| | | | | | | | | | | |
|---|---|---|---|---|---|---|---|---|---|---|
| *Dehydrogenation Performance | | | | | | | | | | |

### Comparative Example 18

The dehydrogenation catalyst of Comparative Example 18 having the following nominal composition, on oxide basis:

| | |
|---|---|
| 9.4% | K₂O |
| 2.2% | MgO |
| 9.9% | CeO₂ |
| 2.5% | MoO₃ |
| 1.9% | CaO |
| 74.1% | Fe₂O₃ |

was prepared as follows:
A mixture of the required amounts of potassium carbonate, magnesium carbonate, cerium carbonate, molybdenum oxide, calcium hydroxide and unhydrated iron oxide was dry blended with a small amount of organic lubricant/poreformer. Water was mulled into the mixture to form an extrudable paste. The paste was formed into cylindrical pellets of 3 mm diameter. The pellets were dried several hours and then calcined at ca. 840°C.

### (Comparative) Examples 19, 20 and Comparative Examples 21 and 22

The catalysts of (Comparative) Examples 19 through 22 were prepared in the manner of the catalyst of Comparative Example 18 except that amounts of a palladium nitrate solution sufficient to produce the target concentrations of Pd, in the calcined catalyst, were added to the water used to prepare the extrudable paste for pelletizing each example catalyst.

The catalysts of Comparative Example 18 and (Comparative) Examples 19 through 22 were tested for dehydrogenation performance in the manner described in Comparative Example 3 except that the range of temperature was 538-593°C. Dehydrogenation performance data are shown in Table VI along with the indicated concentration of Pd. Δ%C is the absolute deviation in % ethylbenzene conversion of the invention example catalysts versus that of the comparative example catalyst. Δ%S₆₀ is the absolute deviation in styrene selectivity at 60% ethylbenzene conversion of the invention example catalysts versus that of the comparative example catalyst. Benzene to toluene, B/T, is the weight ratio of benzene to toluene in the dehydrogenated products.

**TABLE VI**

| Catalyst | Com. Ex 18 | | Example 19 | | Example 20 | | Comparative Example 21 | | Comparative Example 22 | |
|---|---|---|---|---|---|---|---|---|---|---|
| Pd con., wt. % | --- | | 0.0023 | | 0.0050 | | 0.020 | | 0.064 | |
| D.P*. at Temp. °C | Δ%C | B/T | Δ%C | B/T | Δ%C | B/T | Δ%C | B/T | Δ%C | B/T |
| 593 | 0 | .29 | 2.0 | .30 | 1.6 | .30 | 0.9 | .30 | 2.5 | .32 |
| 565 | 0 | .34 | 4.4 | .37 | 4.0 | .36 | 3.9 | .37 | 4.7 | .40 |
| 538 | 0 | .50 | 7.6 | .55 | 6.8 | .50 | 6.3 | .56 | 6.5 | .57 |
| ΔS₆₀ | 0 | | -0.2 | | 0.1 | | -0.2 | | -0.2 | |

| | | | | | | | | | | |
|---|---|---|---|---|---|---|---|---|---|---|
| * Dehydrogenation Performance | | | | | | | | | | |

### Comparative Example 23

The dehydrogenation catalyst of Comparative Example 23 having the following nominal composition, on oxide basis:

| | |
|---|---|
| 9.5% | K₂O |
| 2.2% | MgO |
| 5.0% | CeO₂ |
| 2.5% | MoO₃ |
| 2.0% | CaO |
| 78.8% | Fe₂O₃ |

was prepared as follows:
A mixture of the required amounts of potassium carbonate, magnesium carbonate, molybdenum oxide, calcium hydroxide and unhydrated iron oxide were dry blended with a small amount of organic lubricant/poreformer. The required amount of an aqueous solution of cerium nitrate was mulled into the dry mixture to form an extrudable paste. The paste was formed into cylindrical pellets of 3 mm diameter. The pellets were dried several hours and then calcined (at 600°C).

### Comparative Examples 24 and 25

The catalysts of Comparative Examples 24 and 25 were prepared according to the procedure for the catalyst of Comparative Example 23 except palladium nitrate solution, Comparative Example 24, or dinitrodiamine platinum solution, Comparative Example 25, sufficient to produce a concentration of 640 ppm palladium or 1170 ppm platinum in the respective calcined catalysts, was added to the water used to prepare the extrudable paste for pelletizing the catalysts.

The catalysts of Comparative Example 23 and Comparative Examples 24 and 25 were tested in the manner described in Comparative Example 1. Dehydrogenation performance data are shown in Table VII along with the indicated concentration of Pd or Pt in the example catalysts.

**TABLE VII**

| Catalyst | Com. Ex 1 | Comparative Example 24 | Comparative Example 25 |
|---|---|---|---|
| Promoter | none | Pd | Pt |
| Promoter Concentration, wt. % | --- | 0.064 | 0.117 |
| D.P*. at Temp. °C | Δ%C | Δ%C | Δ%C |
| 600 | 0 | 0.1 | 0.5 |
| 570 | 0 | 5.3 | 4.1 |
| 540 | 0 | 9.5 | 7.3 |
| Δ% S₆₀ | 0 | 0.14 | 0.0 |

### Comparative Example 26

The dehydrogenation catalyst of comparative Example 26 having the following nominal composition, on oxide basis:

| | |
|---|---|
| 9.2% | K2O |
| 2.1% | MgO |
| 9.7% | CeO2 |
| 3.9% | WO3 |
| 1.9% | CaO |
| 73.2% | Fe2O3 |

was prepared as follows:
A mixture of the required amounts of potassium carbonate, magnesium carbonate, ammonium metatungstate, calcium hydroxide and unhydrated iron oxide were blended together with enough water to form an extrudable paste. The paste was formed into cylindrical pellets of 3 mm diameter. The pellets were dried several hours and then calcined at about 840 °C.

### Comparative Example 27

The catalyst of Comparative Example 27 was prepared as follows. A portion of the catalyst prepared as Comparative Example 26 was post impregnated with palladium nitrate, to a level of 0.02% Pd, using standard incipient wetness techniques.

The catalysts of Comparative Example 26 and Comparative Example 27 were tested in the manner described in Comparative Example 18. Dehydrogenation performance data are shown in Table VIII.

**TABLE VIII**

| Catalyst | Com. Ex 26 | | Comparative Example 27 | |
|---|---|---|---|---|
| Pd Concentration, wt. % | --- | | 0.02 | |
| D.P*. at Temp. °C | Δ%C | B/T | Δ%C | B/T |
| 593 | 0 | .16 | 1.6 | .23 |
| 565 | 0 | .16 | 4.7 | .27 |
| 538 | 0 | N/A | 7.0 | .42 |
| ΔS₆₀ | 0 | | -0.05 | |

| | | | | |
|---|---|---|---|---|
| *D.P. = Dehydrogenation Performance | | | | |

### Comparative Example 28

Comparative dehydrogenation catalyst having the following nominal composition, on oxide basis:

| | |
|---|---|
| 9.0% | K2O |
| 2.1% | MgO |
| 9.5% | CeO2 |
| 2.4% | MoO3 |
| 2.1% | CaO |
| 74.8% | Fe2O3 |

was prepared as follows:
A mixture of the required amounts of potassium carbonate, magnesium carbonate, cerium carbonate, molybdenum oxide, calcium hydroxide and unhydrated iron oxide were dry blended with a small amount of organic lubricant/poreformer, mixed with water to form an extrudable paste and then formed into "ribbed" extrusions with a diameter of 2.8 mm (as described in US Patent 5,097,091). The pellets were dried several hours and then calcined (at -840 °C).

### Example 29

The catalyst of Example 29 was prepared as follows. A portion of the catalyst prepared as Comparative Example 28 was post-impregnated with palladium nitrate, to a level of 50 ppm Pd, using standard incipient wetness techniques.

The catalysts of Comparative Example 28 and Example 29 were tested in the manner described in Comparative Example 18. Dehydrogenation performance data are shown in Table IX.

**TABLE IX**

| Catalyst | Com. Example 28 | | Example 29 | |
|---|---|---|---|---|
| Pd concentration wt.% | --- | | 0.005 | |
| D.P.* at Temp °C | Δ%C | B/T | Δ%C | B/T |
| 593 | 0 | 0.26 | 2.5 | 0.31 |
| 565 | 0 | 0.31 | 4.5 | 0.36 |
| 538 | 0 | 0.40 | 8.7 | 0.50 |
| ΔS60 | 0 | | -0.17 | |

| | | | | |
|---|---|---|---|---|
| *D.P. = Dehydrogenation Performance | | | | |

The catalysts of the present invention display improved activity, when compared to its unpromoted counterparts, as evidenced by increased conversion at otherwise identical conditions as shown in Figure 1. This Figure 1 shows the effect of palladium concentration on the catalyst on ethyl benzene conversion with catalysts prepared in the manner of Examples 19-21 (palladium added during pellet formation). Δ%C numbers are the average differences in ethyl benzene conversion at 593°C, 565°C and 538°C between a catalyst with and without various concentration of palladium. This Figure shows improved performance when the concentration of palladium is less than 100 ppm (0.01%). The improved conversion is achieved at no substantial loss in selectivity. Moreover, the by-products formed with palladium or platinum promoted catalysts, of this application, have higher benzene to toluene, B/T, ratio than by-products formed with the non-promoted catalysts, (Comparative) Examples 19 through 22, 27 and 29 vs. comparative Examples 18, 26 and 28, respectively.

Higher activity of the promoted catalysts is evident across the temperature range of the process, as demonstrated by the results of differential tests, (Comparative) Examples 4 and 5, 7 and 8, and 10 through 17 versus Comparative Examples 3, 6 and 9 respectively. By definition the conversions achieved in this type of testing are lower than typical for commercial operation. The advantage of the differential, micro-reactor tests is that it probes the catalyst performance in kinetic regime, free of diffusion interference, thus providing better, fundamental, insight into promoter effects. On the other hand, whole particle, integral, isothermal reactor tests, such as (Comparative) Examples 18 on, reflect better the expected commercial operation of the catalyst. The isothermal tests indicate that the activity increase in promoted catalysts is highest at the lower range of typical operating temperatures in the ethylbenzene to styrene dehydrogenation, Examples 19 through 26. However, this is probably due to diffusional limitations of 3.00 mm pellets and not to lack of promoter effect. Analogous results, of integral tests on smaller size, shorter diffusion path, 2.8 mm ribbed extrusions styrene catalyst demonstrate this point. Higher activity, as evidenced by increased conversion, is observed for Pd-promoted 2.8 mm ribbed extrusions as compared to unpromoted version of the catalyst, Example 29 and Comparative Example 28, respectively. The effect is evident across the temperature range, up to 593°C. Notwithstanding, increased low temperature conversion of Pd promoted 3.00 mm pellets is especially beneficial in an adiabatic system that by default has part of the bed operating at the lower end of the process temperature spectrum. The low temperature operation, for any given set conversion, in addition, brings about extended catalyst life and reduces fouling in the process as compared to the unpromoted catalyst. Advantages resulting from increased conversion of only a few tenths of a percent, not to mention on the order of several percentage points as demonstrated in this invention, are extremely significant in commercial process which may produce many millions of pounds of product per day.

## Claims

1. A dehydrogenation catalyst for an alkylaromatic feed stream containing steam comprising from 40 to 90 weight percent iron oxide calculated as Fe₂O₃, from 5 to 20 weight percent of an alkali metal compound calculated as an alkali metal oxide, from 1 ppm to 100 ppm of a source of noble metal source selected from the group consisting of an elemental noble metals, compounds containing a noble metal and combinations thereof, wherein the noble metal is selected from the group consisting of platinum, palladium and combinations thereof, from 0.5 to 10.0 weight percent of a molybdenum or tungsten compound, calculated as MoO₃ or WO₃ and from 4.0 to 12.0 weight percent of a cerium compound, calculated as CeO₂, wherein all weight percents are based on the total weight of the catalyst.

2. A process for dehydrogenating a mixture of primarily steam and an alkylaromatic feed stream comprising passing the steam/alkylaromatic feed stream over the catalyst of claim 1.

3. Use of a catalyst as defined in claim 1 for the dehydrogenation of ethylbenzene to styrene.

## Patentansprüche

1. Ein Dehydrierungskatalysator für einen alkylaromatischen Zustrom, der Dampf enthält, wobei der Dehydrierungskatalysator 40 bis 90 Gew.-% Eisenoxid, berechnet als Fe₂O₃, 5 bis 20 Gew.-% einer Alkalimetallverbindung, berechnet als ein Alkalimetalloxid, 1 ppm bis 100 ppm einer Edelmetallquelle, ausgewählt aus der Gruppe, bestehend aus elementaren Edelmetallen, edelmetallhaltigen Verbindungen und Kombinationen davon, wobei das Edelmetall ausgewählt ist aus der Gruppe, bestehend aus Platin, Palladium und Kombinationen davon, 0,5 bis 10,0 Gew.-% einer Molybdän- oder Wolframverbindung, berechnet als MoO₃ oder WO₃, und 4,0 bis 12,0 Gew.-% einer Cerverbindung, berechnet als CeO₂, umfasst, wobei alle Gewichtsprozentangaben auf das Gesamtgewicht des Katalysators bezogen sind.

2. Ein Verfahren zur Dehydrierung einer Mischung aus überwiegend Dampf und einem alkylaromatischen Zustrom, umfassend das Leiten von Dampf und alkylaromatischem Zustrom über den Katalysator gemäß Anspruch 1.

3. Verwendung eines Katalysators wie in Anspruch 1 definiert zur Dehydrierung von Ethylbenzol zu Styrol.

## Revendications

1. Catalyseur de déshydrogénation utilisé pour un courant d'alimentation en un alkyle aromatique contenant une vapeur qui comprend de 40 à 90 pour cent en poids d'oxyde de fer (calculés sous forme de Fe₂O₃), de 5 à 20 pour cent en poids d'un composé métallique alcalin (calculés sous forme de l'oxyde d'un métal alcalin), de 1 ppm à 100 ppm d'une source d'un métal précieux sélectionnée dans le groupe consistant en des métaux précieux élémentaires, des composés contenant un métal précieux et des combinaisons de ceux-ci, où le métal précieux est sélectionné dans le groupe consistant en le platine, le palladium et des combinaisons de ceux-ci, de 0,5 à 10,0 pour cent en poids d'un composé du molybdène ou du tungstène (calculés sous forme de MoO₃ ou de WO₃) et de 4,0 à 12,0 pour cent d'un composé de cérium (calculés sous forme de CeO₂), où tous les pourcentages en poids sont calculés en fonction du poids total du catalyseur.

2. Procédé de déshydrogénation d'un mélange composé principalement de vapeur et d'un courant d'alimentation en un alkyle aromatique, qui comprend le passage de la vapeur/du courant d'alimentation en un alkyle aromatique sur le catalyseur de la revendication 1.

3. Utilisation d'un catalyseur tel que défini à la revendication 1 pour la déshydrogénation du benzène éthylique en styrène.
